# EUROPEAN PATENT SPECIFICATION

(11) **EP 3 212 285 B1**
(45) Date of publication and mention of the grant of the patent: **30.01.2019**
(21) Application number: 15787299.5
(22) Date of filing: 10.10.2015
(51) Int. Cl.: A61N 5/06

(54) **A MATTRESS FOR PROVIDING PHOTOTHERAPY TO A SUBJECT**
MATRATZE ZUR VERABREICHUNG VON LICHTTHERAPIE AN EINE PERSON
MATELAS FOURNISSANT UNE PHOTOTHÉRAPIE À UN SUJET

(30) Priority: 28.10.2014 US 201462069625 P
(43) Date of publication of application: 06.09.2017
(73) Proprietor: Koninklijke Philips N.V., 5656 AE Eindhoven (NL)
(72) Inventor: KAESTLE, Siegfried Walter, 5656 AE Eindhoven (NL)
(74) Representative: de Haan, Poul Erik
(86) International application number: PCT/IB2015/057764
(87) International publication number: WO 2016/067137

(56) References cited:
- WO-A2-2008/086375
- US-A1- 2007 100 400
- US-A1- 2007 233 208
- US-A1- 2013 178 919

## Description

### BACKGROUND

### 1. Field

This disclosure relates to providing phototherapy to a subject by embedding light sources in a gel mattress.

### 2. Description of the Related Art

Infants, pre-mature babies, and/or neonates may require phototherapy treatment for jaundice. Jaundice treatment typically incorporate several overhead light sources configured to emit light on jaundiced skin and increase the subject's vitamin D production. The light sources are typically in a rigid overhead location and emit heat in addition to the light radiation, often wasting costly heat and light. With such systems, a doctor or prescribing medical professional may have to make educated guesses regarding the amount of radiation that is being delivered to a subject from the distant light sources (e.g. overhead light sources)

US 2007/0100400 teaches a mattress according to the preamble of claim 1.

### SUMMARY

The invention is defined in claim 1.

One aspect of the present disclosure relates to a mattress that supplies phototherapy to one or more surfaces of a subject's body while the subject is supported on the mattress. In some embodiments, the mattress provides phototherapy to the surfaces of the subject's body and comprises a gel material, a set of light sources, and/or other components. The gel material is formed into and/or held in a generally flattened shape. The flattened shape provides a cushioned support surface. The cushioned support surface is configured to provide support to the subject supported on the mattress. The mattress includes a set of light sources. The light sources are carried by the mattress and configured to emit electromagnetic radiation into the gel material. The electromagnetic radiation emitted into the gel material becomes incident on and passes through the support surface. This configuration provides light therapy to the subject supported on the mattress. The gel material is optically translucent and optically diffusive. This enables the gel material to filter the electromagnetic radiation emitted by the set of light sources. Therapeutically efficacious wavelengths are diffused by the gel material prior to becoming incident on the subject supported on the mattress.

Another aspect of the present disclosure relates to a method for providing phototherapy to a subject with a phototherapy mattress. In some embodiments, the method includes supporting a subject with the mattress. The mattress comprises a gel material formed into and/or held in a generally flattened shape to provide a cushioned support surface for supporting the subject while the subject is supported on the mattress. The method includes emitting electromagnetic radiation from a set of light sources carried by the mattress into the gel material. The electromagnetic radiation becomes incident on the subject after it passes through the support surface. This configuration provides light therapy to the subject supported on the mattress. The electromagnetic radiation emitted by the set of light sources diffuses through the gel material. The mattress is configured such that therapeutically efficacious wavelengths become incident on the subject supported on the mattress.

Yet another aspect of the present disclosure relates to a system configured to provide phototherapy to one or more surfaces of a subject's body in a repose position on a mattress. In some embodiments, the system includes means for supporting a subject comprising a gel material formed into and/or held in a generally flattened shape. This provides a cushioned support surface to a subject supported on the means for supporting. The system includes means for emitting electromagnetic radiation from a set of light sources carried by the means for supporting into the gel material to become incident on and pass through the support surface so as to provide light therapy to a subject supported on the means for supporting. The system includes means for diffusing the electromagnetic radiation such that electromagnetic radiation emitted by the set of light sources and having therapeutically efficacious wavelengths is incident on such a subject supported on the mattress.

These and other objects, features, and characteristics of the system and/or method disclosed herein, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and in the claims, the singular form of "a", "an", and "the" include plural referents unless the context clearly dictates otherwise.

### BRIEF DESCRIPTION OF THE DRAWINGS

FIG. 1 illustrates a mattress system configured for providing phototherapy to a subject.
FIG. 2 illustrates a cross-section of an embodiment of a phototherapy mattress.
FIG. 3 illustrates an embodiment of a phototherapy mattress supporting a subject supported.
FIG. 4 illustrates an embodiment of a phototherapy mattress that is flexible.
FIG. 5 illustrates an embodiment of a phototherapy mattress that has different zones of electromagnetic radiation.
FIG. 6 illustrates a method for providing phototherapy to a subject on a gel mattress.

### DETAILED DESCRIPTION

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

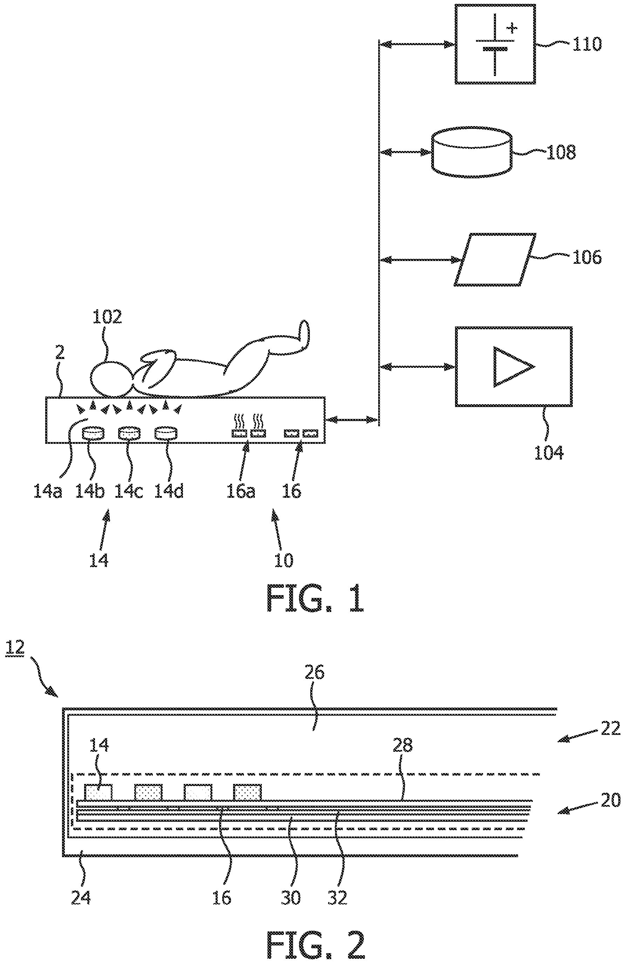

FIG. 1 illustrates a system 10 configured to support a subject 102 while providing phototherapy. System 10 includes a phototherapy mattress 12, a set of light sources 14 (which may include one or more light guides from a light source 14), one or more sensors 16 including thermal sensors 16a, one or more processors 104, a user interface 106, electronic storage 108, a power supply 110, and/or other components.

For illustrative purposes, the treatment of a newborn baby or neonate at home, in a baby ward, and/or in a Neonatal Intensive Care Unit (NICU) is used in this disclosure as an example. Note that the scope of this disclosure is not limited to a specific class or type of patients, such as neonates. Instead, treatment of adults and/or other subjects is contemplated and within the scope of this disclosure. System 10 is not restricted and/or limited to use in and/or with incubators, heat lamps, infant warmers, and/or other devices, though a NICU environment may be a suitable environment for phototherapy. System 10 may be integrated, embedded, incorporated, combined, and/or otherwise operating in conjunction with an incubator, baby warmer, a subject monitoring device (e.g., a patient monitor), a respiratory device, a respiratory monitor, and/or other medical apparatus used for treatment of infants.

Phototherapy mattress 12 may be configured to cover and/or support at least part of subject 102. Phototherapy mattress 12 may include structure to support subject 102. The structure may include cut-outs, holes, three-dimensional shapes, and/or other features to provide support to subject 102. Phototherapy mattress 12 may include diffuse gel, memory foam, and/or other materials to support subject 102. By way of non-limiting example, phototherapy mattress 12 may be and/or include one or more of a mattress, a pad, a blanket, a sleeping bag, flex printed circuit board (FCB), textile layers, and/or other suitable structures to cover and/or support at least part of subject 102.

The set of light sources 14 may be configured to emit electromagnetic radiation 14a. The set of light sources 14 may include multiple light sources, for example a first light source 14b, a second light source 14c, a third light source 14d, and/or other light sources.

One or more sensors 16 may be configured to generate output signals conveying information related to subject 102 while subject 102 is undergoing phototherapy treatment and/or at other times. In some embodiments, subject 102 may be monitored (e.g., by processors 104 described herein) via information generated by one or more sensors 16 and/or other information. Sensors 16 may generate output signals conveying information related to the position, orientation, location, temperature, and/or other information related to subject 102 on phototherapy mattress 12. Sensors 16 may be located within, on, and/or near subject 102 and/or phototherapy mattress 12, and/or at other locations. For example, thermal sensor 16a may be configured to generate output signals conveying temperature information related to subject 102, including but not limited to a skin temperature and/or a temperature at a location in proximity to subject 102. In some embodiments, sensors 16 may be configured to generate output signals conveying information related to an orientation, position, and/or locations of subject 102 with respect to phototherapy mattress 12.

One or more processors 104 are configured to determine the position, orientation, location, temperature, and/or other parameters related to the phototherapy of subject 102 based on the output signals from sensors 16 and/or other information. Processor(s) 104 may determine parameters used to control light sources 14 and/or other components of system 10 based on the output signals of one or more sensors 16, and/or other information.

FIG. 2 illustrates a cross-section of one embodiment of phototherapy mattress 12. Phototherapy mattress 12 may be divided into two or more sections including a layered substrate 20, an envelope 22, and or other sections. Although both sections 20 and 22 in FIG. 2 are illustrated as joined by diffuse gel 26, the two or more sections 20 and 22 may be separable. FIG. 2 is illustrative only and is not intended to be limiting.

Layered substrate 20 may include one or more light sources and/or light guides 14, textile substrates 28, diffuse gel 26, reflective layers 30, an electrical isolation layer 32, and/or other components. Layered substrate 20 provides structural support for phototherapy mattress 12. Layered substrate 20 may provide support, dissipate heat and/or energy, reflect therapeutic electromagnetic radiation, and/or function in other ways. In some embodiments, layered substrate 20 provides support to light source(s) 14 and/or associated light guide(s), sensor(s) 16, subject 102, and/or other components of system 10.

Light sources 14 are configured to emit electromagnetic radiation into diffuse gel 26 to become incident on and pass through mattress 12 so as to provide light therapy to a subject supported on mattress 12 in repose. Light sources 14 may include one or more light emitting diodes (LEDs), organic light emitting diodes (OLEDs), light guides, and/or other sources of light having electromagnetic characteristics configured to provide therapeutic phototherapy. In some embodiments, light source(s) 14 may be arranged to diffuse light through the diffuse gel 26 and onto the body of subject 102. Light source(s) 14 may be configured as a cluster of light sources that have light guides positioned within layered substrate 20. In this configuration, light source(s) 14 may be external to system 10 and the emitted electromagnetic radiation may be guided through one or more light guides within phototherapy mattress 12. The set of light guides may direct the emitted electromagnetic radiation to subject 102 and/or be supported by layered substrate 20. The use of light guides may reduce the heat exposure of light source 14 to subject 102 and may enable a single light source 14 and/or a cluster of light sources 14 to provide diffuse therapeutic electromagnetic radiation to an array of locations on and/or in phototherapy mattress 12.

In some embodiments, light source(s) 14 may be configured to provide electromagnetic radiation having a specific color of light (e.g., light having a specific wavelength range). System 10 may be configured such that the color (wavelength range) is selectable by a user (e.g., via user interface 106 shown in FIG. 1). The selected wavelength and/or color of emitted electromagnetic radiation may have enhanced characteristics and/or qualities related to the phototherapy requirements of subject 102. For example, blue light has particular electromagnetic characteristics that may be therapeutically advantageous and are used to provide specific treatment and/or therapy to subject 102. In some embodiments, electromagnetic radiation with a specific wavelength (e.g. blue light) may be used to treat jaundice and/or hyper-bilirubinemia in an infant. Light source(s) 14, processor 104 (FIG. 1), user interface 106 (FIG. 1) and/or other components of system 10 may be configured to enable and/or facilitate a color and/or wavelength selection of emitted light by a user.

One or more sensors 16 are configured to generate output signals conveying information related to physiological characteristics, position, orientation, temperature, and/or other information related to subject 102. Sensors 16 may include one or more sensors 16 positioned within, on, and/or near phototherapy mattress 12 and/or at other locations. In some embodiments, sensors 16 of system 10 are configured to generate output signals conveying information pertaining to a position, orientation, and/or location of subject 102 on phototherapy mattress 12. In some embodiments, the generated output signals may convey information related to a status of system 10, physiological parameters related to subject 102, presence of subject 102 on mattress 12, environmental parameters, and/or other parameters. For example, sensors 16 may include one or more of a weight sensor, a contact sensor, a heart rate sensor, a respiratory rate sensor, an oxygen sensor, a bilirubin sensor, a tidal volume sensor, an airflow sensor, a pressure sensor, a still-image camera, a video camera, a microphone, and/or other sensors 16. In some embodiments, system 10 may include multiple sensors 16 configured to generate output signals that convey substantially the same information positioned at one or more locations in and/or around mattress 12 (e.g., to facilitate later reduction of variations in measurements and/or to detect measurement gradients by processor 104 shown in FIG. 1, for example).

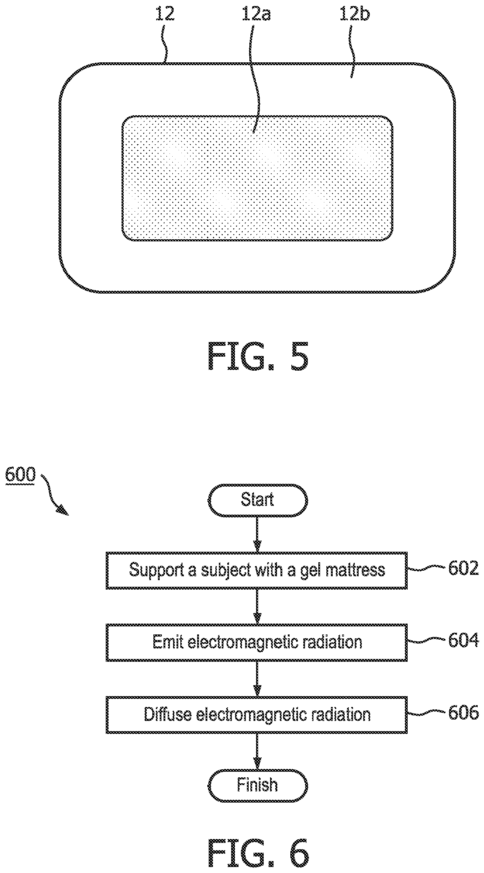

In some embodiments, the output signals form sensors 16 may convey information that is used (e.g., by processor 104 shown in FIG. 1) to determine zones of the phototherapy mattress 12 (e.g., which light sources 14) that should be controlled to emit higher and/or lower levels of incident phototherapy. In some embodiments, the absence of a signal from sensor 16 may indicate a zone of phototherapy mattress 12 that should emit reduced (e.g. zero) electromagnetic radiation (zones are further described below related to FIG. 5).

One or more thermal sensors 16a of system 10 are configured to generate output signals conveying information related to one or more temperatures. The temperatures may include one or more temperatures in and/or near phototherapy mattress 12, one or more temperatures of subject 102, and/or other temperatures. For example, a thermal sensor 16a may be configured to generate output signals conveying information regarding a skin temperature of subject 102. For example, sensors 16a may include a temperature sensor and/or infra-red (IR) sensor in proximity to the skin of subject 102 and may be configured to generate output signals conveying information regarding the skin temperature of subject 102.

Sensors 16 are configured such that the output signals and/or information conveyed by the output signals from sensors 16 may be transmitted to processor 104, user interface 106, electronic storage 108, and/or other components of system 10. This transmission may be via wires and/or wireless.

As described above, phototherapy mattress 12 may include one or more textile substrates 28. Textile substrates 28 may be and/or include one or more layers including a reflector layer 30, an electrical isolation layer 32, a light source and/or light guide layer (e.g., that includes light sources and /or light guides 14), and/or other layers. In some embodiments, textile substrates 28 may be printed using a flex printed circuit board (FCB).

Reflector layer 30 is configured to enhance the amount of therapeutic electromagnetic radiation diffused through gel 26 by reflecting radiation from light sources 14 toward subject 102. Reflector layer 30 may also reduce the amount of glare phototherapy mattress 12 may emit into the eyes of caretakers. Reflective layer 30 may decrease the amount of light required by the light sources 14 and thus reduce the heat emitted from the light sources 14. This may have the effect of lowering the overall temperature of phototherapy mattress 12. In some embodiments, the flex printed circuit board (FCB) is finished with a white cover mask and/or film to act as a reflector.

Electrical isolation layer 32 may provide electrical isolation for light source(s) 14, sensor(s) 16, and/or other electronic components within phototherapy mattress 12. In some embodiments, electrical isolation layer 32 may be formed as and/or include a thin film and/or other features. Electrical isolation layer 32 may be surrounded by diffuse gel 26 and/or other components of phototherapy mattress 12. In some embodiments, electrical isolation layer 32 may be a membrane, flex printed circuit board (FCB), and/or textile layer that provides electrical isolation. For example, a light source 14 and/or a sensor 16 may require electrical power. Electrical isolation layer 32 may facilitate providing these components with power while isolating the electronic charge of the power provided from diffuse gel 26 and/or subject 102.

In some embodiments, envelope 22 may cover, surround, and/or otherwise envelope layered substrate 20. Envelope 22 may include a flexible and/or wipeable cover 24, diffuse gel 26, and/or other components. In some locations, cover 24 may be soft and/or thin (for example) to provide a comfortable surface of mattress 12 for subject 102 (FIG. 1) to lie on. In some locations, cover 24 may be hard and/or thick (for example) to provide structural support to the components of system 10. Diffuse gel 26 is included within cover 24 and may provide comfort to subject 102, diffuse electromagnetic radiation, reduce heat and/or provide other functionality as described herein. Envelope 22 may be removably coupled with layered substrate 20. This may facilitate washing envelope 22, disposing of envelope 22, replacing envelope 22, and/or other activities, while retaining layered substrate 20. This configuration may also enable limited numbers of layered substrates 20 to provide phototherapy to multiple subjects 102 without requiring the transfer of a subject 102 to a different phototherapy mattress 12. For example, an individual subject 102 may remain on his/her individual envelope 22 while a single layered substrate 20 section is rotated to each subject 102 in order to provide the individual subjects 102 their prescribed regimen of phototherapy.

As described above, in some embodiments, cover 24 is wipeable. In some embodiments, it may be necessary to use mattress 12 and/or cover 24 to treat several different subjects 102 on a hospital ward. Cover 24 would typically be cleaned and disinfected between uses for individual subjects. The cleaning/disinfection solutions used on cover 24 may be caustic. Cover 24 may be configured to be cleaned easily and/or be resistant to caustic cleaning solutions, heat, friction, and/or have other properties to assist the cleaning of cover 24. In some embodiments, cover 24 may be a soft non-absorbent cover 24.

Diffuse gel 26 may be included in layered substrate 20, envelope 22, and/or in other components of phototherapy mattress 12. Diffuse gel 26 is configured to be optically translucent, and/or otherwise configured to diffuse light from light sources 14. Diffuse gel 26 is optically diffusive such that electromagnetic radiation emitted by the set of light sources 14 and/or light-guides will have therapeutically efficacious wavelengths and be diffused by diffuse gel 26 prior to becoming incident on subject 102 supported on phototherapy mattress 12. Diffuse gel 26 decreases the uniformity of exposure of subject 102 to the light provided by light sources 14 and increases the therapeutic effect of electromagnetic radiation therapy.

In some embodiments, diffuse gel 26 provides support to subject 102. In some embodiments, the viscosity of diffuse gel 26 may be selected (e.g., at manufacture) to enhance (e.g., make mattress 12 feel more firm) and/or reduce (e.g., make mattress 12 feel softer) structural support. For example, a highly viscous diffuse gel 26 material may be selected to provide a firm support to subject 102. Alternatively, a low viscosity of diffuse gel 26 may be selected to permit more comfort to subject 102.

In some embodiments, viscous diffuse gel 26 and/or layered substrate 20 may be used without cover 24. In this embodiment, diffuse gel 26 may be selected so as to provide comfortable support to subject 102, diffuse electromagnetic radiation, be resistant to cleaning solutions, diffuse heat, and/or provide other functionality as described herein.

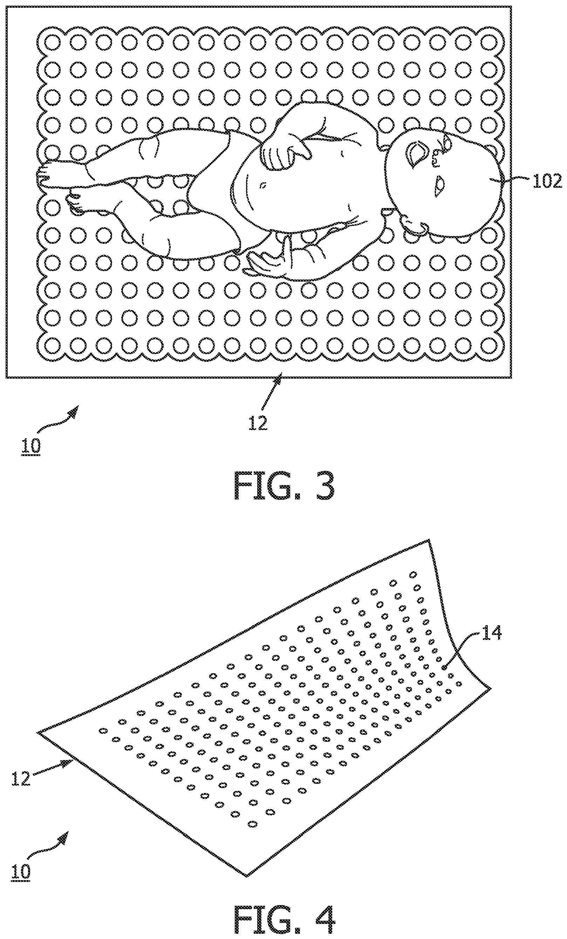

FIG. 3 illustrates a subject 102 on one example embodiment of a phototherapy mattress 12. As shown in FIG. 3, system 10 provides comfortable support to subject 102 supported on phototherapy mattress 12. In FIG. 3, system 10 is shown providing therapeutic blue light to subject 102 while subject 102 is in a comfortable repose position on mattress 12. This is not intended to be limiting. As described herein, system 10 may be configured to provide light of any therapeutic wavelength. FIG. 3 illustrates light emitting diodes (LEDs) light sources 14 embedded in epoxy to mechanically stabilize light sources 14. In some embodiments transparent and/or reflective cups may be glued over light sources 14 (e.g. LEDs) to mechanically stabilize light sources 14.

FIG. 4 illustrates another embodiment of system 10. FIG. 4 illustrates the flexibility of phototherapy mattress 12 (e.g., due to the selected material properties of at least gel 26 shown in FIG. 2). In FIG. 4, blue organic light emitting diode (OLEDs) light sources 14 are illustrated clustered at various locations within mattress 12.

In some embodiments, a particular zone, region, section, area, and/or portion of phototherapy mattress 12 may emit more phototherapy (e.g. electromagnetic) radiation than other zones. FIG. 5 illustrates two zones 12a, 12b of phototherapy mattress 12 that emit different levels of phototherapy radiation. In the example shown in FIG. 5, a first zone 12a emits more electromagnetic radiation than a second zone 12b. First zone 12a may include more densely packed light sources (e.g., light sources 14 shown in FIG. 1) and second zone 12b may include less light sources and/or no light sources at all, for example. In some embodiments, the light sources in a zone may be independently controlled (e.g., by processor 104 based on output signals from sensors 16). The shape, configuration, orientation, number of zones, and/or other information depicted in FIG. 5 is not intended to be limiting and is illustrative only.

In some embodiments, different zones, regions, sections, areas, zones, and/or portions of phototherapy mattress 12 may include different materials and/or have different phototherapy characteristics. For example, in some embodiments, system 10 may be configured such that a particular zone, region, section, area, and/or portion of phototherapy mattress 12 may include a material, component, and/or layer having a known (e.g., determined at manufacture) heat transfer coefficient (e.g. thermal conductance per unit area). As another example (as described above), a first zone of phototherapy mattress 12 (e.g., zone 12a) may provide a first level (e.g., intensity and/or wavelength of light provided by light sources 14) of phototherapy; a second zone of phototherapy mattress 12 (e.g., zone 12b) may provide a second level of phototherapy, and so forth.

In some embodiments, the first level of phototherapy is greater than the second level of phototherapy. In some embodiments, the first level, second level, and/or other levels of phototherapy may be zero. For example, a part of phototherapy mattress 12 on which a subject (e.g., subject 102 in FIG. 1) is supported may have a higher level of phototherapy than at least part of the remainder of phototherapy mattress 12.

In some embodiments, the size and/or shape of the zones of phototherapy are controlled by processor 104, user interface 106, and/or other components of system 10 based on the output signals from one or more sensors 16 (FIG. 1), information entered and/or received via user interface 106, prior phototherapy of a subject, and/or other information. For example, the subject may be determined (e.g., by processor 104 shown in FIG. 1) to be smaller than phototherapy mattress 12 (e.g., based on output signals from sensors 16). In this example, processor 104 (FIG. 1) may be configured to determine a first zone in which light generated would be incident to the subject and a second zone in which generated light would not be incident to the subject. Processor 104 may control the first zone to provide more phototherapy (e.g., higher intensity light) to the subject than the second zone (e.g., in which there may be no light provided at all) based on the determination. In some embodiments, the subject's head and/or other body parts may extend beyond an end of phototherapy mattress 12. System 10 may be configured to provide phototherapy based on the part of the subject's body touching mattress 12. In some embodiments, system 10 is configured to limit the size of a zone delivering a higher level of phototherapy for heat management purposes and/or to provide more intense phototherapy treatment via specific areas of mattress 12.

In some embodiments, system 10 may be configured such that zones of phototherapy mattress 12 may be configured to provide therapeutic light to a subject while preventing incident light and/or glare from reaching the eyes of a caregiver which may reduce headaches, distractions, and/or other issues associated with the delivery of phototherapy to a subject for a caregiver of the subject.

In some embodiments, system 10 may be configured such that phototherapy is controlled by zone to accommodate users of different sizes and/or a single subject requiring prolonged phototherapy. For example, in some embodiments, prolonged therapeutic light incident on a subject's head may result in elevated core body temperatures of the subject. System 10 may be configured to adjust the amount of light incident on the subject's head over time. As another example, the size of the subject may change (e.g. grow). This may be particularly true of neonates and infants that may require prolonged phototherapy. The control of the zones of phototherapy mattress 12 may be manually accomplished by a caregiver (e.g., via user interface 106) and/or automated (e.g., via processor 104) based on information conveyed by the output signals from one or more sensors 16 (FIG. 1), and/or other information.

Returning to FIG. 1, power supply 110 may be configured to provide the power to operate and/or activate one or more light sources 14. In some embodiments, power supply 110 may provide power to one or more sensors 16, processor(s) 104, user interface 106, electronic storage 108, and/or other components of system 10.

User interface 106 of system 10 may be configured to provide an interface between system 10 and a user (e.g., a subject, a caregiver, a healthcare provider, a therapy decision-maker, a medical professional etc.) through which the user can provide information to and receive information from system 10. This enables data, results, and/or instructions and any other communicable items, collectively referred to as "information," to be communicated between the user and system 10. An example of information that may be conveyed to a user is a report detailing the changes in monitored temperature throughout a period during which subject 102 is present on mattress 12. Examples of interface devices suitable for inclusion in user interface 106 include a keypad, buttons, switches, a keyboard, knobs, levers, a display screen, a touch screen, speakers, a microphone, an indicator light, an audible alarm, and a printer. Information may be provided to a user by user interface 106 in the form of auditory signals, visual signals, tactile signals, and/or other sensory signals, or any combination thereof

It is to be understood that other communication techniques, either hard-wired or wireless, are contemplated herein as user interface 106. For example, in some embodiments, user interface 106 may be integrated with a removable storage interface provided by electronic storage 108. In this example, information is loaded into system 10 from removable storage (e.g., a smart card, a flash drive, a removable disk, etc.) that enables the user(s) to customize the implementation of system 10. Other exemplary input devices and techniques adapted for use with system 10 as user interface 106 include, but are not limited to, an RS-232 port, RF link, an IR link, a USB connection, modem (telephone, cable, Ethernet, internet or other). In short, any technique for communicating information with system 10 is contemplated as user interface 106.

Electronic storage 108 of system 10 includes electronic storage media that electronically stores information. The electronic storage media of electronic storage 108 may include one or both of system storage that is provided integrally (i.e., substantially non-removable) with system 10 and/or removable storage that is removably connectable to system 10 via, for example, a port (e.g., a USB port, a FireWire port, etc.) or a drive (e.g., a disk drive, etc.). Electronic storage 108 may include one or more of optically readable storage media (e.g., optical disks, etc.), magnetically readable storage media (e.g., magnetic tape, magnetic hard drive, floppy drive, etc.), electrical charge-based storage media (e.g., EPROM, EEPROM, RAM, etc.), solid-state storage media (e.g., flash drive, etc.), and/or other electronically readable storage media. Electronic storage 108 may store software algorithms, information determined by processor 104, information received via user interface 106, and/or other information that enables system 10 to function properly. For example, electronic storage 108 may record and/or store one or more measured temperatures, and/or other information. Electronic storage 108 may be a separate component within system 10, or electronic storage 108 may be provided integrally with one or more other components of system 10 (e.g., processor 104).

Processor 104 of system 10 is configured to provide information processing capabilities in system 10. As such, processor 104 includes one or more of a digital processor, an analog processor, a digital circuit designed to process information, an analog circuit designed to process information, and/or other mechanisms for electronically processing information. Although processor 104 is shown in FIG. 1 as a single entity, this is for illustrative purposes only. In some embodiments, processor 104 includes a plurality of processing units.

Processor 104 may be configured to control light source(s) 14. Processor may control the color, intensity, and/or other properties of light source(s) 14. Processor 104 may control light sources 14 and/or other components of system 10 based on information entered and/or selected via user interface 106, information stored in electronic storage 108, information received from one or more sensors 16 (including thermal sensors 16a, sensors configured to convey information relating to the operation of system 10 and/or the subject 102, and/or other sensors 16), and/or other information. Processor(s) 104 may determine parameters based on the output signals of sensors 16 and use the determined parameters to control light sources 14 and/or other components of system 10. Processor 104 may control a size, a shape, a therapy intensity and/or other characteristics of one or more zones of mattress 12 as described herein.

In some embodiments, system 10 may include a power/control port that is configured to removably couple light sources 14 with power and/or control devices external to system 10. In some embodiments, this port may be facilitate communication between processor 104 and sensors 16, light source(s) 14, and/or other components of system 10.

FIG. 6 illustrates a method 600 for providing phototherapy to a subject. The operations of method 600 presented below are intended to be illustrative. In some embodiments, method 600 may be accomplished with one or more additional operations not described, and/or without one or more of the operations discussed. Additionally, the order in which the operations of method 600 are illustrated in FIG. 6 and described below is not intended to be limiting.

In some embodiments, method 600 may be implemented in (and/or using) one or more processing devices (e.g., a digital processor, an analog processor, a digital circuit designed to process information, an analog circuit designed to process information, and/or other mechanisms for electronically processing information). The one or more processing devices may include one or more devices executing at least some of the operations of method 600 in response to instructions stored electronically on an electronic storage medium. The one or more processing devices may include one or more devices configured through hardware, firmware, and/or software to be specifically designed for execution of one or more of the operations of method 600.

At an operation 602, a subject is supported by a mattress comprising gel material formed into and/or held in a generally flattened shape. The gel material provides a cushioned support surface to a subject supported on the mattress. In some embodiments, operation 602 is performed by a phototherapy mattress the same as or similar to phototherapy mattress 12 (shown in FIG. 1 and described herein).

At an operation 604, electromagnetic radiation is emitted from a set of light sources carried by the mattress. The electromagnetic radiation passes through the gel material and support surface to become incident on and provide light therapy to the subject supported on the mattress. In some embodiments, operation 604 is performed by a light source the same as or similar to light source 14 (shown in FIG. 1 and described herein).

At an operation 606, electromagnetic radiation is diffused. The electromagnetic radiation emitted by the set of light sources and having therapeutically efficacious wavelengths is incident on such a subject supported on the mattress. In some embodiments, operation 606 is performed by a gel material the same as or similar to diffuse gel 26 (shown in FIG. 2 and described herein).

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the system(s) and/or method(s) of this disclosure have been described in detail for the purpose of illustration based on what is currently considered to be the most practical and preferred embodiments, it is to be understood that such detail is solely for that purpose and that the disclosure is not limited to the disclosed embodiments, but is intended to cover modifications that are within the scope of the appended claims. For example, it is to be understood that the present disclosure contemplates that, to the extent possible, one or more features of any implementation can be combined with one or more features of any other implementation.

## Claims

1. A mattress (12) for providing phototherapy to a subject (102), the mattress comprising:
a gel material (26) formed into and/or held in a generally flattened shape to provide a cushioned support surface to the subject while the subject is supported on the mattress;
a set of light sources (14) carried by the mattress, the set of light sources configured to emit electromagnetic radiation into the gel material to become incident on and pass through the support surface so as to provide light therapy to such a subject supported on the mattress; and
**characterized in that**
the gel material is optically translucent and optically diffusive such that electromagnetic radiation emitted by the set of light sources and having therapeutically efficacious wavelengths is diffused by the gel material prior to becoming incident on such a subject supported on the mattress.

2. The mattress of claim 1, wherein the mattress includes an envelope (22) forming a cavity that holds the gel, the envelope including a first membrane at the support surface, the first membrane being transparent or translucent.

3. The mattress of claim 2, wherein a reflective layer (30) is placed on an opposite side of the envelope from the support surface, the reflective layer formed by a second membrane of the envelope.

4. The mattress of claim 1, further comprising a power and/or control port configured to removably couple the light sources with power and/or control devices.

5. The mattress of claim 1, wherein the mattress comprises zones of electromagnetic radiation configured to accommodate users of different sizes or the development of a single user.

## Patentansprüche

1. Matratze (12) zum Bereitstellen einer Lichttherapie für eine Person (102), die Matratze umfassend:
ein Gelmaterial (26), das in einer im Allgemeinen abgeflachten Form geformt und/oder gehalten wird, um der Person eine gepolsterte Auflagefläche bereitzustellen, während die Person auf der Matratze aufliegt;
einen Satz von Lichtquellen (14), die von der Matratze getragen werden, wobei der Satz von Lichtquellen konfiguriert ist, um elektromagnetische Strahlung in das Gelmaterial zu emittieren, um auf die Auflagefläche aufzutreffen und durch diese hindurchzugehen, um eine Lichttherapie für eine solche auf der Matratze aufliegende Person bereitzustellen; und
**dadurch gekennzeichnet, dass**
das Gelmaterial optisch lichtdurchlässig und optisch diffundierend ist, so dass elektromagnetische Strahlung, die von dem Satz von Lichtquellen emittiert wird und therapeutisch wirksame Wellenlängen aufweist, durch das Gelmaterial diffundiert wird, bevor sie auf eine solche auf der Matratze aufliegende Person trifft.

2. Matratze nach Anspruch 1, wobei die Matratze eine Hülle (22) beinhaltet, die einen Hohlraum bildet, der das Gel hält, wobei die Hülle eine erste Membran an der Auflagefläche beinhaltet, wobei die erste Membran transparent oder lichtdurchlässig ist.

3. Matratze nach Anspruch 2, wobei eine reflektierende Schicht (30) auf einer der Auflagefläche gegenüberliegenden Seite der Hülle angeordnet ist, wobei die reflektierende Schicht durch eine zweite Membran der Hülle gebildet wird.

4. Matratze nach Anspruch 1, weiter umfassend einen Stromversorgungs- und/oder Steueranschluss, der konfiguriert ist, um die Lichtquellen abnehmbar mit Stromversorgungs- und/oder Steuervorrichtungen zu koppeln.

5. Matratze nach Anspruch 1, wobei die Matratze Zonen elektromagnetischer Strahlung umfasst, die konfiguriert sind, um Benutzer verschiedener Größen oder die Entwicklung eines einzelnen Benutzers aufzunehmen.

## Revendications

1. Matelas (12) pour fournir une photothérapie à un sujet (102), le matelas comprenant :
un matériau de gel (26) formé en et/ou maintenu dans une forme généralement aplatie pour fournir une surface de support rembourrée au sujet pendant que le sujet est supporté sur le matelas ;
un ensemble de sources de lumière (14) supportées par le matelas, l'ensemble de sources de lumière étant conçu pour émettre un rayonnement électromagnétique dans le matériau de gel afin de devenir incident et de passer à travers la surface de support de manière à fournir une luminothérapie à un tel sujet appuyé sur le matelas ; et
**caractérisé en ce que**
le matériau de gel est optiquement translucide et optiquement diffusif de sorte qu'un rayonnement électromagnétique émis par l'ensemble de sources de lumière et présentant des longueurs d'ondes thérapeutiquement efficaces est diffusé par le matériau de gel avant de devenir incident sur un tel sujet supporté par le matelas.

2. Matelas selon la revendication 1, dans lequel le matelas comprend une enveloppe (22) formant une cavité qui contient le gel, l'enveloppe comprenant une première membrane au niveau de la surface de support, la première membrane étant transparente ou translucide.

3. Matelas selon la revendication 2, dans lequel une couche réfléchissante (30) est placée sur un côté de l'enveloppe opposé à la surface de support, la couche réfléchissante étant formée par une seconde membrane de l'enveloppe.

4. Matelas selon la revendication 1, comprenant en outre un port d'alimentation et/ou de commande configuré pour coupler de manière amovible les sources de lumière avec des dispositifs d'alimentation et/ou de commande.

5. Matelas selon la revendication 1, dans lequel le matelas comprend des zones de rayonnement électromagnétique configurées pour accueillir des utilisateurs de différentes tailles ou pour le développement d'un utilisateur unique.
